# EUROPÄISCHE PATENTSCHRIFT

(11) **EP 3 532 839 B1**
(45) Veröffentlichungstag und Bekanntmachung des Hinweises auf die Patenterteilung: **02.12.2020**
(21) Anmeldenummer: 17797553.9
(22) Anmeldetag: 26.10.2017
(51) Int. Cl.: G01N 33/08

(54) **ERZEUGUNG EINES LOCHS IN EINEM VOGELEI ZUR GESCHLECHTSBESTIMMUNG DES VOGELEIES**
PRODUCING A HOLE IN A BIRD EGG FOR DETERMINING THE SEX OF THE BIRD EGG
GÉNÉRATION D'UN TROU DANS UN OEUF D'OISEAU POUR DÉTERMINER LE SEXE D'UN OEUF D'OISEAU

(30) Priorität: 26.10.2016 DE 102016013155
(43) Veröffentlichungstag der Anmeldung: 04.09.2019
(73) Patentinhaber: Technische Universität Dresden, 01069 Dresden (DE)
(72) Erfinder: STEINER, Gerald, 08340 Schwarzenberg (DE); PREUSSE, Grit, 01445 Radebeul (DE); KOCH, Edmund, 01307 Dresden (DE); GALLI, Roberta, 01309 Dresden (DE); SCHNABEL, Christian, 01099 Dresden (DE)
(74) Vertreter: Rauschenbach, Marion
(86) Internationale Anmeldenummer: PCT/EP2017/077507
(87) Internationale Veröffentlichungsnummer: WO 2018/078046

(56) Entgegenhaltungen:
- WO-A1-2017/017277
- JP-A- S6 455 131
- US-A1- 2014 158 050
- ROBERTA GALLI ET AL: "In Ovo Sexing of Domestic Chicken Eggs by Raman Spectroscopy", ANALYTICAL CHEMISTRY, Bd. 88, Nr. 17, 11. August 2016 (2016-08-11), Seiten 8657-8663, XP55378439, US ISSN: 0003-2700, DOI: 10.1021/acs.analchem.6b01868

## Beschreibung

Die Erfindung betrifft ein Verfahren zur Positionierung einer Messstelle an mindestens einem Blut führenden Gefäß eines geöffneten Vogeleies zur nachfolgenden Geschlechtsbestimmung des Vogeleies.

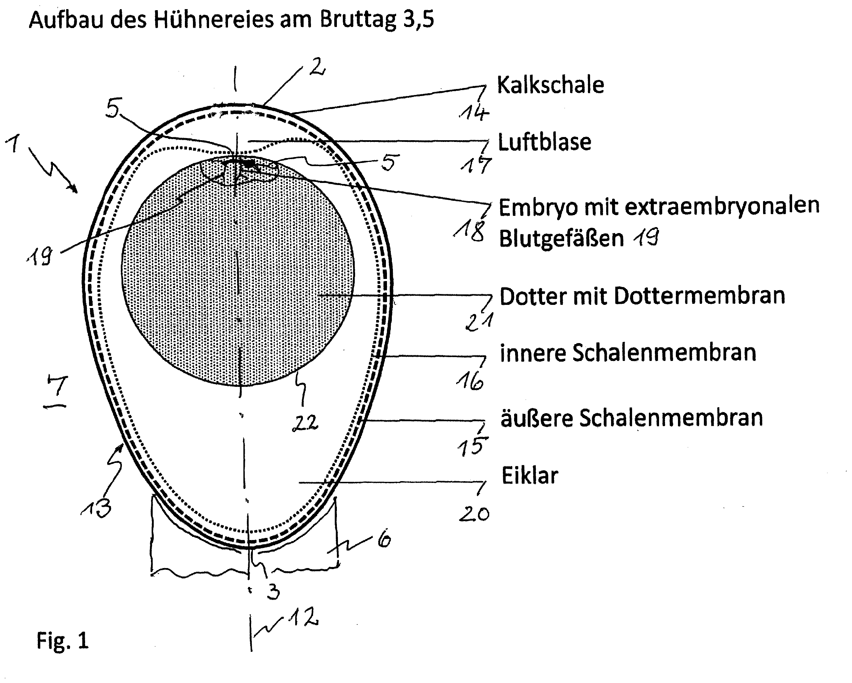

Im Allgemeinen weist gemäß Fig. 1 ein Hühnerei 1 am 3,5. Bruttag eine Eischale 13, bestehend aus einer Kalkschale 14 mit anhaftender äußerer Schalenmembran 15 und innerer Schalenmembran 16, eine Luftkammer 17, einen Embryo 18 mit extraembryonalen Blutgefäßen 19, ein Eiklar 20 und ein Dotter 21, welches von der Dottermembran 22 umgeben ist, auf. Die Eischale 13 stellt für das Ei 1 während der gesamten Inkubation eine wichtige Schutzbarriere dar. Die Kalkschale 14 schützt den Eiinhalt während der Brutzeit vor mechanischen Einwirkungen. An der Innenseite der Kalkschale 14 liegen Schalenmembranen an, bestehend aus der äußeren Schalenmembran 15 und der inneren Schalenmembran 16. Die Schalenmembranen verhindern das Eindringen von Mikroorganismen und regulieren das Milieu im Inneren des Eies 1, beispielsweise das Flüssigkeitsgleichgewicht. In Abhängigkeit davon, zu welchem Zeitpunkt und wie stark die Schutzbarriere durch ein sogenanntes "Fenstern" der Eischale 13 durchbrochen wird, können die Entwicklung des Embryos 18 und die Schlupfraten stark beeinflusst werden, wobei unter der Fensterung das Einbringen eines Loches 8 in die Eischale 13 verstanden wird.

So sinkt die Schlupfrate nicht bebrüteter Eier nach der Fensterung durch die gesamte Eischale dramatisch, die Entfernung ausschließlich eines Kalkschalenfensters unter Erhalt der Schalenmembran beeinträchtigt die Schlupfrate jedoch nicht, wie in der Druckschrift J. Brake, et al.: Egg Handling and Storage, 1997, Poultry science 76; S. 144-151 beschrieben ist.

Ein Verfahren zur Geschlechtsbestimmung von Vogeleiern ist gemäß der Zwischenveröffentlichung WO 2017/017277 A1 offenbart, wobei ein Loch am stumpfen Pol des Eies erzeugt wird, welches die innere Membran innerhalb der Luftkammer unversehrt lässt. Anschließend wird im Bereich des Lochs ein Blutgefäß unterhalb der inneren Membran registriert, das Blut mittels Raman-Spektroskopie untersucht und das Messergebnis für die Geschlechtsbestimmung ausgewertet.

Gemäß R. Galli, et al. Analytical Chemistry 2016, 88, S. 8657-8663 ist ein Verfahren und eine Vorrichtung zur Geschlechtsbestimmung von Vogeleiern offenbart, wobei ein Loch am spitzen Pol des Eies erzeugt wird, anschließend ein Blutgefäß im Bereich des Lochs registriert wrid, das Blut darin mittels Raman-Spektroskopie untersucht und das Messergebnis für die Geschlechtsbestimmung ausgewertet wird.

Laut dem Stand der Technik benötigen Verfahren zur Geschlechtsbestimmung zu einem sehr frühen Zeitpunkt der Inkubation, also in einem Zeitraum zwischen dem Beginn der Bebrütung und dem siebenten Bebrütungstag vor Einsetzen des Schmerzempfinden, ein Fenster 8 in der Eischale 13, um einen Zugang für optische Messungen zu schaffen, wie in den Druckschriften DE 10 2014 010 150 A1: Verfahren und Vorrichtung zur Ramanspektroskopischen in-ovo Geschlechtsbestimmung von befruchteten und bebrüteten Hühnereier, DE 10 2016 005 974.5: Verfahren und Vorrichtung zur Einstellung des Laserfokus eines Anregungslasers in blutdurchflossenen Gefäßen für optische Messungen zur Bestimmung des Geschlechtes von Vogeleiern, und DE 10 2016 004 051.3: Verfahren und Vorrichtung zur optischen in-ovo Geschlechtsbestimmung von befruchteten und bebrüteten Vogeleiern, beschrieben ist.

Ein Nachteil der genannten Verfahren in den angegebenen Druckschriften besteht darin, dass in Abhängigkeit vom Zeitpunkt der Untersuchung und von der Lochgröße die Schlupfrate bei diesen Verfahren durch die Fensterung beeinträchtigt werden kann, sofern das Fenster 8 sowohl durch die Kalkschale 14 als auch durch die beiden Schalenmembranen 15, 16 hindurch geschaffen wird. Das ist, bei Einsatz eines Lasers gemäß der in oben genannten Druckschriften vorgegebenen Parameter, an jeder Stelle des Hühnereies 1 der Fall.

Die Fensterung kann prinzipiell überall am Vogelei erfolgen, bevorzugt erfolgt sie pollastig am spitzen Pol.

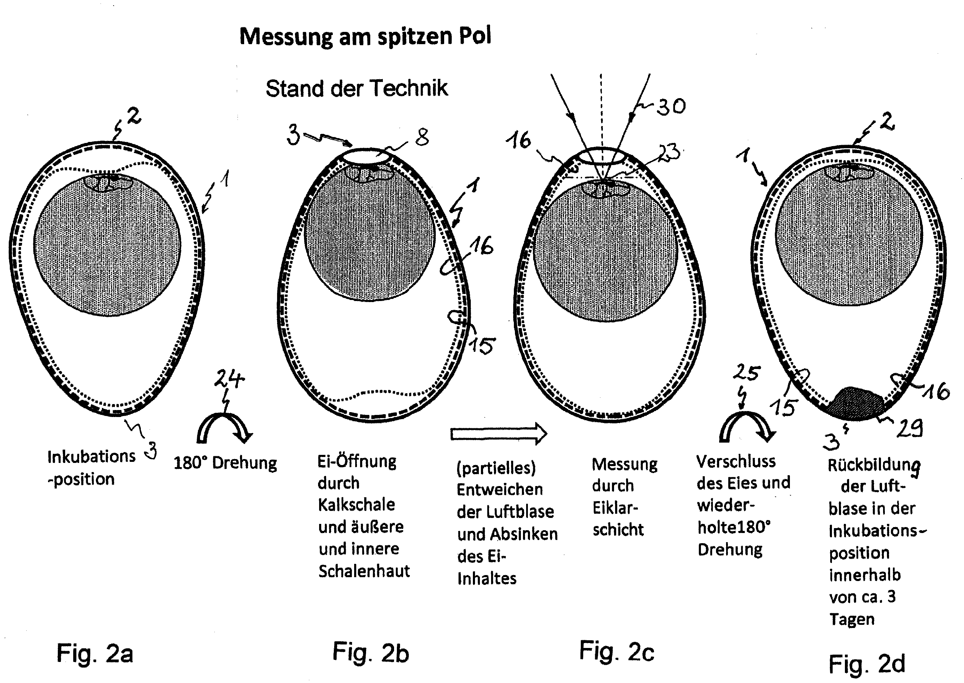

In einer kommerziellen Brüterei werden die Eier gemäß Fig. 2 in vertikaler Position ausschließlich mit dem spitzen Pol 3 nach unten ausgebrütet. Der stumpfe Pol 2 ist nach oben gerichtet, da sonst keine Embryonalentwicklung erfolgt. Bei der Schaffung eines Loches 8 am spitzen Pol 3 gemäß Fig. 2b werden die Eier 1 vor der Eiöffnung und der nachfolgenden Geschlechtsbestimmung um 180° gewendet. Da äußere Schalenmembran 15 und Kalkschale 14 miteinander verhaftet sind, beinhaltet gewöhnlich die Schaffung eines Loches 8 am spitzen Pol 3 sowohl die Entfernung der Kalkschale 14 als auch meist der gesamten Schalenmembran 15, 16 innerhalb der gesetzten Perforation. Das partielle oder vollständige Entweichen der Luft aus der Luftblase 17 führt gemäß Fig. 2c zu einem Absinken des Eiinhaltes, was ein kontinuierliches Nachverfolgen der Blutgefäße 19 in vertikaler z-Richtung der Eiachse 12 während der Registrierung der Rückstreustrahlung erfordert. Nach der Geschlechtsbestimmung werden gemäß Fig. 2d die Eier verschlossen und erneut um 180° gewendet. Die Fig. 2a, 2b, 2c, 2d zeigen die herkömmliche optische Messung am spitzen Pol 3 eines bebrüteten Hühnereies 1 und die notwendigen Drehungen 24 und 25. In Fig. 2b und Fig. 2c und Fig. 2c ist die innere Schalenmembran 16 im Bereich des Lochs defekt bzw. versehrt, gelocht und/oder entfernt worden. In Fig. 2d ist ein Verschluss 29 angebracht, der das komplette Loch mit den gelochten Schalenmembranen 15 und 16 verschließt.

Der Nachteil besteht in der hohen Zeitverzögerung durch die notwendigen Drehungen, bis eine Messung durchgeführt werden kann.

Somit befindet sich der Embryo 18 beim Setzen der Laserperforation am spitzen Pol 3 gemäß Stand der Technik nach vorheriger Drehung des Eies 1 direkt unter der Eischale 13, wie in Fig. 2b gezeigt ist. Eine Verletzung des Embryos 18 oder der extraembryonalen Blutgefäße 19 durch Wärmeschädigung während des Einbringens der Laserperforation ist durch die Variabilität der Kalkschalendicke nicht ausgeschlossen.

Bei einer Eiöffnung am spitzen Pol 3 führt die unter Umgebungsdruck wirkende Schwerkraft (aufgrund der Masse von überwiegend Eigelb und Eiklar) auf die Luftkammer 17 zu einem partiellen oder vollständigen Entweichen der Luft. Das bedingt eine vertikale Bewegung des Blutgefäßes 19 (z-Richtung/Ei-Achse 12) in der Größenordnung der Höhe der Luftblase 17 vor bzw. während der Registrierung der Rückstreustrahlung. Da sich dabei das mit der Laserstrahlung 30 bestrahlte Blutgefäß 19 aus dem Laserfokus heraus bewegen kann, ist ein Gefäßtracking erforderlich.

Der Erfindung liegt deshalb die Aufgabe zugrunde, ein Verfahren zur Positionierung einer Messstelle an mindestens einem Blut führenden Gefäß eines geöffneten Vogeleies zur nachfolgenden Geschlechtsbestimmung des Vogeleies anzugeben, das derart geeignet ausgebildet ist, dass zumindest Drehungen der bebrüteten Eier und ein Gefäßtracking bei geöffnetem Vogelei vermieden werden können.

Die Aufgabe wird mit den Merkmalen der unabhängigen Patentansprüche gelöst, wobei die abhängigen Patentansprüche vorteilhafte Ausgestaltungen der Erfindung angeben.

In dem Verfahren zur Positionierung einer Messstelle an mindestens einem Blut führenden Gefäß eines geöffneten Vogeleies zur nachfolgenden Geschlechtsbestimmung des Vogeleies mit einer Erzeugung eines Lochs an dem Vogelei, das zumindest einen stumpfen Pol und einen spitzen Pol sowie eine Eischale mit einer inneren Schalenmembran und einer äußeren, der Kalkschale des Vogeleies näher liegenden Schalenmembran aufweist, wird das Vogelei zur Durchführung zumindest einer das Blut betreffenden optischen Messung geöffnet, wobei die Erzeugung des Lochs am stumpfen Pol des Vogeleies mittels einer Locherzeugungseinheit durchgeführt wird, wobei das Loch die Kalkschale und die äußere, eine Luftkammer mit der inneren Schalenmembran bildenden Schalenmembran betrifft und die innere Schalenmembran unversehrt bleibt, wobei im Bereich des Lochs am stumpfen Pol unterhalb der unversehrten inneren Schalenmembran zumindest ein Blutgefäß registriert und mittels einer einfallenden vorgegebenen Strahlung das Blut angeregt wird, dessen durch die unversehrte innere Schalenmembran hindurch geführte Rückstreustrahlung gemessen, detektiert und für eine Geschlechtsbestimmung ausgewertet wird, wobei in einer xy-Ebene drehbare/ kippbare Achsen mittels einer motorgesteuerten Kippvorrichtung, die mit einer zentralen Steuereinheit in Verbindung steht, verdreht werden, sodass bei Abweichung des Embryos aus der zentralen Eiachse der abweichende Embryo in den unmittelbaren Bereich der Eiachse zentriert gebracht wird und ein minimaler Abstand zwischen der Wölbung der inneren Schalenmembran und dem Embryo erreicht wird.

Dabei wird die Kalkschale mit anhaftender äußerer Schalenmembran geöffnet und das Blut zumindest des Blutgefäßes spektroskopisch untersucht und es bleibt während der spektroskopischen optischen Messsignalaufnahme durch zumindest die innere Schalenmembran der Eischale hindurch die innere Schalenmembran unversehrt.

Im Rahmen der Erfindung wird unter der Registrierung eines Blutgefäßes das Auffinden und Auswählen des mindestens einen, für die Anregung des Blutes und die weitere Geschlechtsbestimmung, vorgesehenen extraembryonalen oder embryonalen Blutgefäßes im geöffneten Vogelei verstanden, wobei bei der Registrierung des Blutgefäßes auch die Fokussierung der optischen Einheit auf das registrierte Blutgefäß durchgeführt wird (Fokussierung in x, y, z Ebene). Vor und während der Messung kann eine Temperierung der Eihalterung und der Messumgebung des Eies mit einer zugeordneten Einheit zur Temperierung durchgeführt werden.

Nach der Detektion der gewählten Rückstreustrahlung wird das Loch im Pol verschlossen und die Bebrütung des als weiblich geschlechtsbestimmten Eies wird fortgesetzt.

Bei der Fensterung am stumpfen Pol des Eies im Bereich der Luftblase ergeben sich folgende Vorteile:
- Sofern von der in allen kommerziellen Brütereien verwendeten vertikalen Positionierung des Eies mit dem nach oben gerichteten stumpfen Pol ausgegangen wird, wird das Ei vor und nach der Geschlechtsbestimmung nicht gewendet bzw. verdreht werden.
- Bedingt durch Dichteunterschiede befindet sich der Embryo einschließlich der extraembryonalen Blutgefäße in den ersten Inkubationstagen immer am höchsten Punkt des Eiinneren, d.h. im Bereich des stumpfen Pols. Bei dem Setzen einer Laserperforation in vertikaler Brutposition, d.h. erfindungsgemäß mit stumpfem Pol nach oben, befindet sich der Embryo unterhalb der inneren Schalenmembran. Zwischen der Kalkschale und der inneren Schalenmembran sorgt die Luftblase dafür, dass das Verletzungsrisiko für den Embryo beim Setzen der Laserperforation erheblich reduziert ist.

- Die Eiöffnung bzw. die Locherzeugung beinhaltet die Entfernung der Kalkschale und ausschließlich der äußeren Schalenmembran innerhalb der Perforation, der Embryo bleibt unterhalb der inneren Schalenmembran vor äußeren Umgebungseinflüssen und einem möglichen Keimeintrag geschützt. Mittels dieses sehr viel weniger invasiven Verfahrens wird erfindungsgemäß eine deutliche Verbesserung der Schlupfrate bewirkt.
- Der Erhalt der gesamten inneren Schalenmembran führt weiterhin zum weitestgehenden Erhalt der Lageverhältnisse von Eidotter, Eiklar und Luft innerhalb des Eies. Während der Bebrütung entsteht im Ei ein geringer Unterdruck.
- Bei der Eiöffnung am stumpfen Pol tritt eine durch die Druckänderung bedingte vertikale Bewegung der Blutgefäße nicht auf, da sich keine kompressierbare Luft unterhalb von Dotter und Eiklar befindet. Durch die Änderung der auf die innere Schalenmembran wirkenden Kräfte bei Änderung des Druckes von leichtem Unterdruck auf den Umgebungsdruck mit Erzeugung des Eiloches am stumpfen Pol kommt es nur zu einer minimalen Änderung der Wölbung der inneren Schalenmembran. Diese Druckänderung führt dazu, dass der Embryo mit den extraembryonalen Blutgefäßen für die Dauer der Registrierung der Rückstreustrahlung reversibel an der inneren Schalenmembran fixiert wird, was eine horizontale Bewegung der Blutgefäße in x,y -Richtung verhindert. Somit entfällt das Nachführen der Blutgefäße oder des Objektives (Gefäßtracking) sowohl in vertikaler als auch in horizontaler Richtung. Eine Bewegung der Blutgefäße aus dem Laserfokus, die durch eine Volumenverkleinerung des Eiinneren bedingt sein könnte, tritt nicht auf, da das Ei keiner starken Temperaturabsenkung während der Registrierung der Rückstreustrahlung ausgesetzt ist.

Die erfindungsgemäße Vorrichtung zur Positionierung einer Messstelle an mindestens einem Blut führenden Gefäß eines geöffneten Vogeleies innerhalb einer Eihorde zur nachfolgenden Geschlechtsbestimmung des Vogeleies bei Durchführung des vorgenannten Verfahrens umfasst zumindest
- eine Locherzeugungseinheit zur Erzeugung eines Lochs am stumpfen Pol des Vogeleies,
- eine optische Messeinrichtung zur Messung der das Blut betreffenden Rückstreustrahlung,
- zwei in einer xy-Ebene drehbare/kippbare Achsen, die mit der Eihorde in Verbindung stehen, wobei die Achsen von Dreh/Kippsignalen bedient werden,
- eine motorgesteuerte Kippvorrichtung, die mit den Achsen in Verbindung steht, und
- eine zentrale Steuerungseinheit, die mit der motorgesteuerten Kippvorrichtung in Verbindung steht,
wobei die Achsen derart verdreht werden, dass bei Abweichung des Embryos aus der zentralen Eiachse der abweichende Embryo in den unmittelbaren Bereich der Eiachse zentriert gebracht wird und ein minimaler Abstand zwischen der Wölbung der inneren Schalenmembran und Embryo erreicht wird.

Die Verbindungen zwischen der motorgesteuerten Kippvorrichtung und den Achsen sind zumindest mittels der Leitungen und die Verbindungen zwischen der motorgesteuerten Kippvorrichtung und der zentralen Steuerungseinheit sind zumindest mittels der Leitungen ausgebildet.

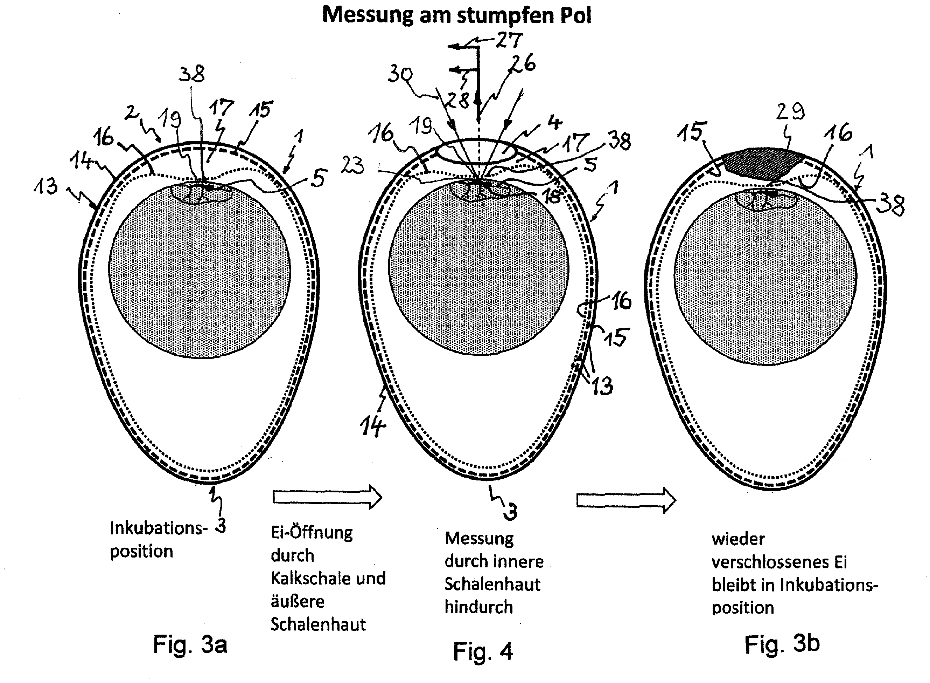

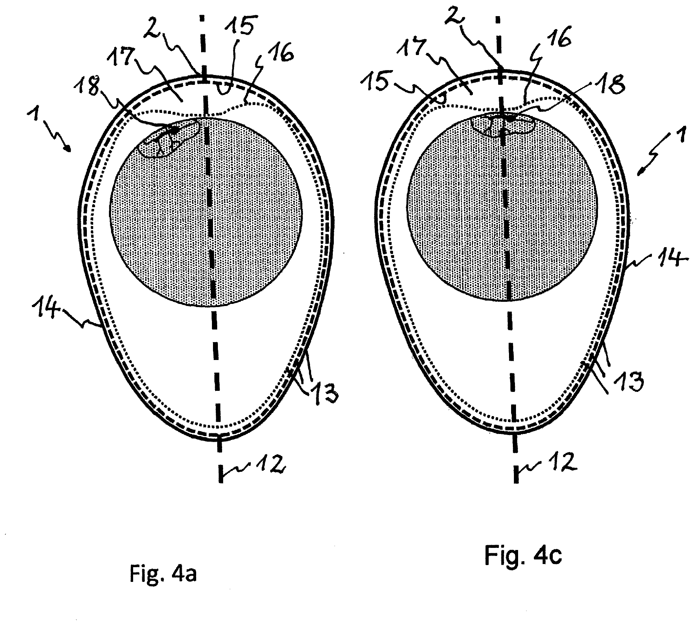

Vor der Erzeugung des Loches 4 am stumpfen Pol 2 können der Embryo 18 und die Blutgefäße 19, 5 zentriert werden. Die Dichteunterschiede im Ei 1 in den ersten Tagen der Bebrütung führen dazu, dass sich der Embryo 18 einschließlich der extraembryonalen Blutgefäße 19 immer am höchsten Punkt des Eiinneren befindet, das heißt im Bereich des stumpfen Pols 2. Das bedeutet, dass sich bei Bebrütung in vertikaler Brutposition, wie sie in kommerziellen Brütereien angewendet wird, der Embryo 18 und die Blutgefäße 19 direkt unter der inneren Schalenmembran 16 am stumpfen Pol 2 des Eies 1 befinden, und zwar üblicherweise im Zentrum des Eies 1, wie in Fig. 4c gezeigt ist. In einigen Fällen kann der Embryo 18 durch die natürliche Variabilität der Eier 1, wie in Fig. 4a gezeigt ist, eine leicht seitlich verschobene Position zur vertikalen Eiachse 12 einnehmen und wird in dieser Position durch schwache Adhäsionskräfte zur inneren Schalenmembran 16 festgehalten. In Fig. 4c ist der Normalfall dargestellt.

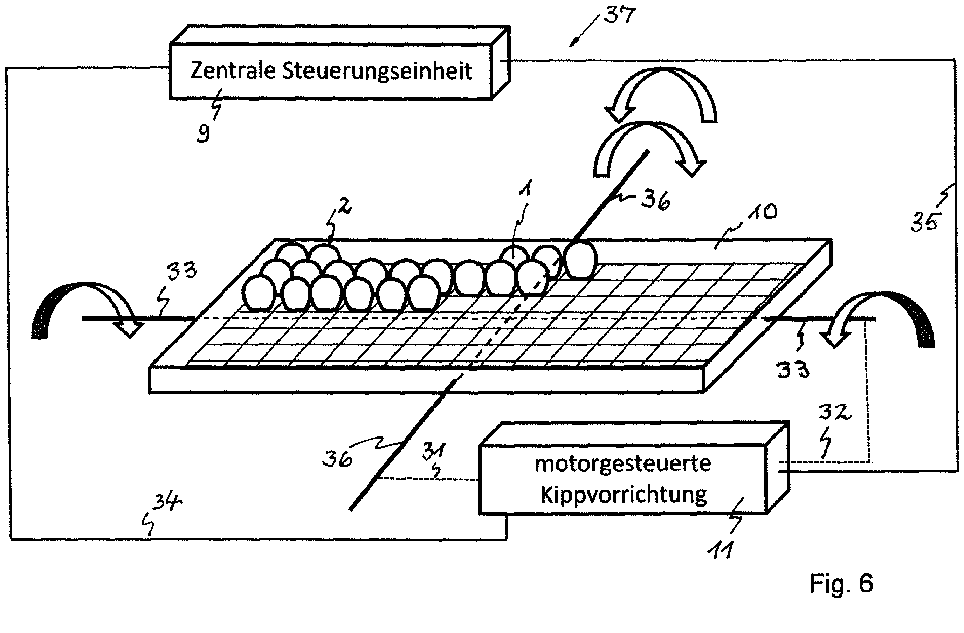

Um die Prozentzahl der Eier, in denen der Embryo 18 zentriert unterhalb der inneren Schalenmembran 16 schwimmt, zu erhöhen, wird erfindungsgemäß eine Embryo-Zentriereinheit 8 mit einer Kippvorrichtung 11 gemäß Fig. 6 eingesetzt. Damit wird erreicht, dass sich für jede Messung der Geschlechtsbestimmung zumindest ein Blutgefäß 19 mit möglichst großem Durchmesser im Messfeld 23 befindet.

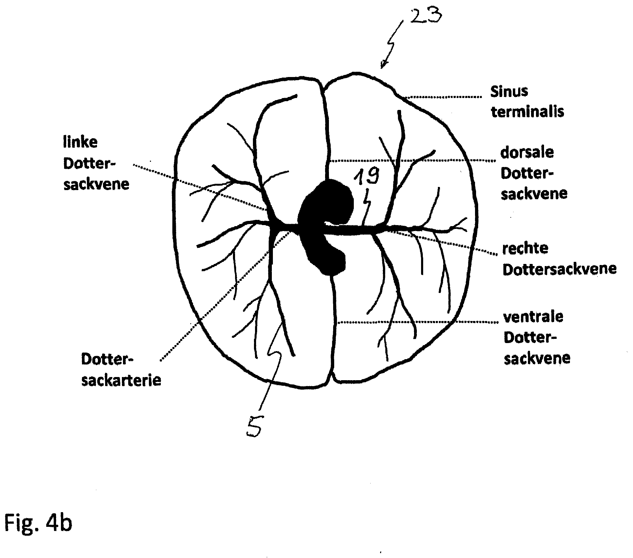

Das extraembryonale Blutgeflecht 19 am vierten Bruttag beschreibt eine annähernd kreisförmige Fläche 23 als Messstelle, wie in Fig. 4b dargestellt ist. Prinzipiell ist die Messung in jedem Blutgefäß 19, 5, 18 dieser Kreisfläche 23 durchführbar, jedoch ist in größeren Blutgefäßen 19 die Signalintensität größer und damit die Dauer der spektroskopischen, das Blut betreffenden Messung kürzer. Die extraembryonalen Blutgefäße 19 im Zentrum, das bedeutet in direkter Verbindung mit dem embryonalen Blutkreislauf, weisen den größten Durchmesser auf. In der Umgebung des sinus terminalis wird der Durchmesser der feiner verzweigten Blutgefäße 5 dagegen kleiner.

Das Zentrieren des Embryos 18 findet bevorzugt vor der Erzeugung des Schalenfensters am stumpfen Pol 2 des Eies 1 statt, da der Embryo 18 mit den ex-traembryonalen Blutgefäßen 19 nach der Öffnung 4 schwach an der inneren Schalenmembran 16 haftet, wobei dieses Anhaften reversibel ist.

In der erfindungsgemäßen Vorrichtung 37 mit der Zentrierungssteuereinheit/zentralen Steuereinheit/Embryozentriereinheit 9 und Kippvorrichtung 11 werden die Eier 1 einzeln oder in einer Eihorde 10 durch eine Bewegung in einem Winkel von mindestens 10° bis 90°, bevorzugt zwischen 30° - 60°, jeweils in x- und y- Richtung gekippt oder kippend rotiert, wobei unter einer Eihorde 10 die in kommerziellen Brütereien verwendeten oft mehrere 20zigen bis 50zigen Eihalterungen 6 in den Brutschränken verstanden wird. Die Geschwindigkeit des Durchlaufs der Horde 10 und der einstellbare Neigungs-Winkel werden so festgelegt, dass die Embryonalentwicklung nicht beeinträchtigt ist.

Die Schaffung eines Lochs 4 in der Kalkschale 14 des Vogeleies 1 erfolgt mittels einer Locherzeugungseinheit bevorzugt mit einem Laser und erfindungsgemäß am stumpfen Pol 2 mit einem Durchmesser von 3 mm bis 18mm, bevorzugt zwischen 8 mm und 15 mm.

Eine Gefäßdetektion kann mittels einer optischen Einheit zur Fokussierung in ein einzelnes Blutgefäß (Fokussierung in x,y,z Ebene) durch die innere Schalenmembran 16 hindurch erfolgen. Allerdings ist die Transparenz der inneren Schalenmembran 16 in den variablen Vogeleiern, auch abhängig von der Rasse, unterschiedlich stark ausgeprägt.
Deshalb konnte herausgefunden werden, dass es für die Geschlechtsbestimmung der Vogeleier notwendig ist, zur Detektion der Blutgefäße nicht ausschließlich die Fließbewegung der Erythrozyten als Schärfeindikator zu nutzen, sondern erfindungsgemäß zusätzliche optische Signale, wie beispielsweise intensitätsbezogene Messsignale oder Signale der optischen Cohärenztomographie (OCT), einzusetzen. Aus diesem Grund wird erfindungsgemäß die Anregung des Blutes durch einfallende Strahlung 30 und die Registrierung der Rückstreustrahlung (Ramanstrahlung 27 und/oder Fluoreszenzstrahlung 28) mit einer optischen Einheit realisiert.

Die Registrierung der Blutgefäße und die optische Anregung des Blutes werden dadurch realisiert, dass der Fokus der einfallenden Anregungsstrahlung in ein Blutgefäß positioniert und ein von einem Laser generiertes Signal genutzt wird. Dieses Signal ist vorzugsweise so fokussiert, dass die Dimension des Laserspots zum Durchmesser der Blutgefäße vergleichbar oder kleiner ist. Die Laserwellenlänge wird so gewählt, dass die Anregung der einfallenden Strahlung ein Signal vom Blut erzeugt, welches zur Anregungslaserwellenlänge spektral verschoben ist.

Die Intensität der Rückstreustrahlung wird in dem Teilbereich des Spektrums gemessen, der blutspezifische Informationen enthält. Das vom Blut in den Gefäßen erhaltene Signal unterscheidet sich dabei in der Intensität und/oder den spektralen Eigenschaften zu Signalen, die von Ei-Strukturen außerhalb der Blutgefäße erhalten werden.

Die korrekte Fokusposition entspricht den Koordinaten, bei denen das Intensitätssignal ein Maximum erreicht. Dabei kann die Intensität der Fluoreszenz oder die Intensität und das Profil einer Ramanbande oder die Intensitäten und Profile mehrerer Ramanbanden einer Blutkomponente das blutspezifische intensitätsbezogene Signal bilden, welches erfindungsgemäß zur Fokusfindung durch die innere Schalenmembran genutzt wird. Der Laser, dessen Fokus zur Positionierung verwendet wird, kann derselbe Laser sein, der auch zur Anregung des geschlechtsspezifischen Raman- und/oder Fluoreszenzsignales genutzt wird. Der Laser, der zur Positionierung des Laserfokus in das Blutgefäß eingesetzt wird, kann auch ein zur Anregung des geschlechtsspezifischen Signales unterschiedlicher Laser sein, der nur zum Zweck der Positionierung des Laserfokus genutzt wird. Der Positionierungslaser wird in diesem Fall so ausgewählt, dass ein in der Intensität sehr starkes blutspezifisches (aber nicht zwingend geschlechtsspezifisches) Signal erzeugt wird, welches mit einfachen, preiswerten und schnellen Detektionssystemen registriert werden kann. Möglich ist auch einen Positionierungslaser einzusetzen, der wesentlich preiswerter im Vergleich zu Laserquellen ist, die zur Anregung des geschlechtsspezifischen Signales des Blutes genutzt werden.

Dabei ist zu berücksichtigen, dass die eingeschränkte Transparenz der inneren Schalenmembran nicht nur eine erfindungsgemäße Anpassung der Methode zur Positionierung des Laserfokus in das Blutgefäß erfordert, sondern dass auch die Intensität des geschlechtsspezifischen Signales, welches zur Geschlechtsbestimmung der Eier akquiriert wird, beeinflusst wird.

Die innere Schalenmembran des Eies ist ein inhomogenes Medium, welches sowohl die einfallende Laserstrahlung als auch das generierte geschlechtsspezifische Signal, das vom Blut emittiert wird, streut. Um die Variationen in der Transparenz der inneren Schalenmembran unter den Eiern korrigieren zu können, muss ein Parameter genutzt werden, mit dem die Intensität des geschlechtsspezifischen Signales korrigiert und der Intensitätsverlust aufgrund der reduzierten Membrantransparenz kompensiert wird.

Möglich ist, dass mehrere Laser für die Registrierung der Blutgefäße und die Anregung des Blutes eingesetzt werden. So kann ein Laser zur Anregung von geschlechtsspezifischen Raman- und Fluoreszenzsignalen im Bereich 790nm und 1050 nm eingesetzt werden, während zweiter grüner oder blauer Laser dafür eingesetzt wird, um eine intensive blutspezifische Hämoglobinfluoreszenz zwischen 550nm und 750 nm anzuregen. Die Intensität dieser Fluoreszenz kann mit Photodioden gemessen und zur Positionierung des Laserfokus im Blutgefäß genutzt werden.

Die Vorteile des erfindungsgemäßen Verfahrens sind folgende:
- Hühnereier werden in kommerziellen Brütereien ausschließlich in vertikale Position ausgebrütet. Dabei muss herkömmlich der spitze Pol nach unten positioniert werden, da es sonst zu keiner Embryonalentwicklung kommt. Eine spektroskopische Geschlechtsbestimmung am spitzen Pol erfordert somit ein zweimaliges Wenden der Eier vor und nach der spektroskopischen Geschlechtsbestimmung (wie in den Figuren 2a, 2b, 2c, 2d gezeigt). Bei der erfindungsgemäßen Geschlechtsbestimmung am stumpfen Pol 2 entfällt dagegen dieses zweimalige Wenden, was sich für die Embryonalentwicklung als positiv erweist.
- Beim aus dem Stand der Technik bekannten Öffnen der Eier am spitzen Pol 3 kommt es durch die Druckunterschiede im Ei 1 zum partiellen oder vollständigen Entweichen der Luft aus der Luftblase, was eine vertikale Bewegung der Blutgefäße bedingt. Durch den erfindungsgemäßen Erhalt der inneren Schalenmembran 16 bleiben dagegen die Blutgefäße 5, 18,19 fixiert.
- Zwischen dem Embryo 18 und der Eischale 13 im Bereich der Laserperforation befindet sich die Luftkammer 17. Somit ist erfindungsgemäß während des Setzens der Laserperforation und des Abhebens des Schalendeckels vom übrigen Teil des Eies 1 das Verletzungsrisiko des Embryos 18 bzw. der extraembryonalen Blutgefäße 19 verringert.

Ein wesentlicher Vorteil des erfindungsgemäßen Verfahrens ist es, dass es eine spektroskopische Geschlechtsbestimmung zu einem sehr frühen Zeitpunkt der Bebrütung unter minimal invasiven Bedingungen ermöglicht. Damit wird erreicht, dass die Schlupfraten unbeeinflusst bleiben.

Bezüglich der erfindungsgemäßen Vorrichtung ist die Eihorde 10 mittels zwei senkrecht zueinander angeordneter Dreh-/Kippachsen 33 und 36 gelagert. Über die zugehörigen Leitungen 31 und 32 werden die Dreh-/Kippachsen 33 und 36 mit Dreh-/Kippsignalen versorgt. Eine zentrale Steuerungseinheit oder Embryo-Zentriereinheit 9 versorgt die motorgesteuerte Kippvorrichtung 11 mit Dreh-/Kippsignalen, die sie an die Achsen 33 und 36 weitergibt.

Die Vorrichtung 37 zur Positionierung einer Messstelle 23 an mindestens einem Blut führenden Gefäß 5, 18, 19 eines geöffneten Vogeleies 1 innerhalb einer Eihorde 10 zur nachfolgenden Geschlechtsbestimmung des Vogeleies 1 umfasst zumindest eine Locherzeugungseinheit zur Erzeugung eines Lochs (4) am stumpfen Pol (2) des Vogeleies (1), eine optische Messeinrichtung zur Messung der das Blut betreffenden Rückstreustrahlung (26), zwei in einer xy-Ebene drehbare/kippbare Achsen 33, 36, die mit der Eihorde 10 in Verbindung stehen, wobei die Achsen 33, 36 von Dreh/Kippsignalen bedient werden, eine motorgesteuerte Kippvorrichtung 11, die mit den Achsen 33, 36 in Verbindung steht, und eine zentrale Steuerungseinheit 9, die mit der motorgesteuerten Kippvorrichtung 11 in Verbindung steht, wobei die Achsen 33, 36 derart verdreht werden, dass bei Abweichung des Embryos 18 aus der zentralen Eiachse 12 der abweichende Embryo 18 in den unmittelbaren Bereich der Eiachse 12 zentriert gebracht wird und ein minimaler Abstand zwischen der Wöbung 38 der inneren Schalenmembran 16 und des Embryos 18 erreicht wird.

Die Verbindungen zwischen der motorgesteuerten Kippvorrichtung 11 und den Achsen 33 und 36 sind mittels der Leitungen 31, 32 und die Verbindungen zwischen der motorgesteuerten Kippvorrichtung 11 und der zentralen Steuerungseinheit 9 sind mittels der Leitungen 34, 35 ausgebildet.

Die Locherzeugungseinheit ist derart auf die Perforation des Schalendeckels eingestellt, dass während der Perforation und während des nachfolgenden Abhebens des Schalendeckels zur Ausbildung des Lochs 8 die innere Schalenmembran 16 nicht beschädigt wird.

Die Erfindung wird mittels zwei Ausführungsbeispiele anhand von Zeichnungen erläutert.

Dabei zeigen:
- Fig. 1: eine schematische Darstellung des Aufbaus eines Hühnereis am 3,5-ten Bruttag gemäß dem Stand der Technik,
- Fig. 2: schematische Darstellungen der Schrittfolge zur Durchführung einer Messung am spitzen Pol eines Hühnereies nach dem Stand der Technik, wobei
- Fig. 2a: eine Inkubationsposition,
- Fig. 2b: eine Eiöffnung durch die Kalkschale sowie äußere Schalenmembran und innere Schalenmembran hindurch,
- Fig. 2c: eine Messung an embryonalen oder extraembryonalen Blutgefäßen durch die Eiklarschicht hindurch und
- Fig. 2d: die Rückbildung der Luftblase in die Inkubationsposition innerhalb von drei Tagen zeigen,
- Fig. 3a: Inkubationsposition zur nachfolgenden Durchführung einer Messung am stumpfen Pol eines Hühnereies,
- Fig. 3b: ein wieder verschlossenes, als weiblich bestimmtes Hühnerei, das in der Inkubationsposition bleibt,
- Fig. 4: eine Ei-Öffnung durch die Kalkschale und äußere Schalenmembran hindurch mit einer optischen, das Blut betreffenden Messung durch die unversehrte innere Schalenmembran hindurch, wobei das Blut eines unmittelbar unterhalb der inneren Schalenmembran liegenden Blutgefäßes gemessen wird,
- Fig. 4a: eine schematische Darstellung eines Eies durch die natürliche Variabilität der Eier mit einer leicht seitlich verschobenen Position zur vertikalen Eiachse,
- Fig. 4b: ein extraembryonales Blutgeflecht am vierten Bruttag in Form einer annähernd kreisförmigen Fläche,
- Fig. 4c: eine schematische Darstellung eines Eies durch ein Festhalten des Embryogeflechts in der Position zur vertikalen Ei-Achse durch schwache Adhäsionskräfte zur inneren Schalenmembran,
- Fig. 5: zwei ausgewertete Intensitäts(normiert)/relative Wellenzahl-Kurven beispielsweise für fünf Hühnereier mit männlichem Geschlecht (durchgezogene Linie) und für fünf Hühnereier mit weiblichem Geschlecht (gestrichelte Linie) nach einer optischen Messung der Rückstreustrahlung des Blutes durch die unversehrte innere Schalenmembran hindurch und
- Fig. 6: eine schematische Darstellung einer Eihorde in Verbindung mit einer Kippvorrichtung zum Kippen um mindestens zwei festgelegte Achsen und einer Zentriersteuereinheit.

### Ausführungsbeispiel 1

Der im ungeöffneten Ei frei bewegliche Embryo 18 wird durch zweimaliges Kippen eines Vogeleies 1 um einen Winkel von etwa 40° in x- und y- Richtung unter der inneren Schalenmembran 16 zentriert. Eine Öffnung mit einem Durchmesser von 15mm wird am stumpfen Pol 2 am Tag 3,5 der Inkubation eingebracht. Ein extraembryonales Blutgefäß 19 wird unter grüner LED Beleuchtung mit einem Spektralbereich von 500nm - 550nm und mittels einer Kamera durch die innere Schalenmembran 16 hindurch selektiert. Ein CW-Laserstrahl 30 der Laserstrahlenquelle (Leistung 200mW; A=785nm) wird auf das extraembryonale Blutgefäß 19 mittels eines Objektives fokussiert und das Blut angeregt.

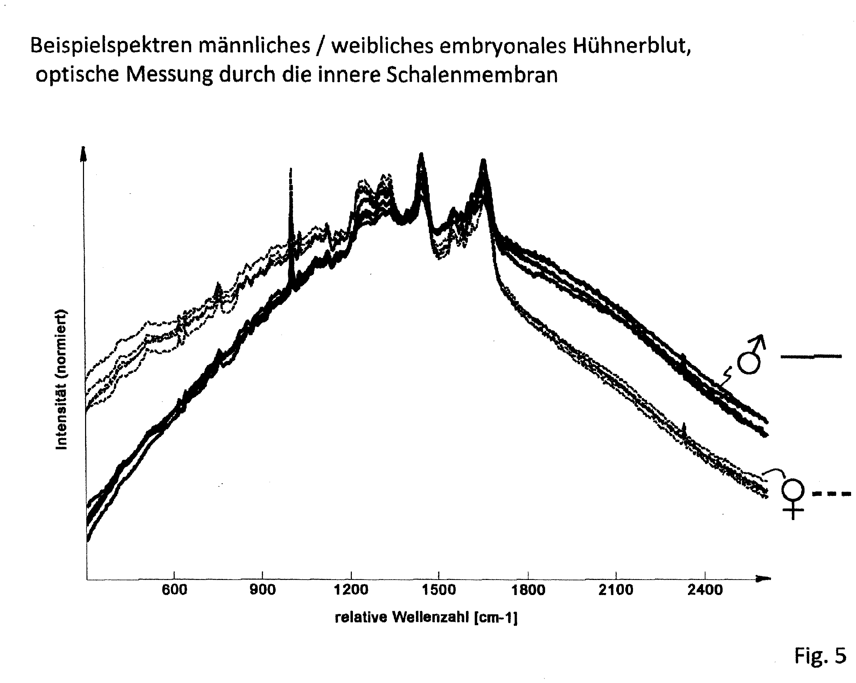

Nachfolgend wird die Registrierung der Rückstreustrahlung 26 einschließlich der Fluoreszenzstrahlung 28 und die Auswertung in einer Auswerteeinheit durchgeführt. Als durchgeführtes Verfahren zur Vorbehandlung der registrierten Spektren zur Geschlechtsbestimmung wird die Vektornormierung eingesetzt, bei der eine Normierung der Intensität unter Bezug auf die relative Wellenzahl durchgeführt wird (Fig. 5).

Zusätzlich werden die Eihalterung 6 und die Messumgebung 7 temperiert, um die Faltenbildung der inneren Schalenmembran 16 bei der Eiöffnung zu vermeiden und damit Signalverluste durch Streuung der Rückstreustrahlung 26 an der inneren Schalenmembran 16 zu minimieren. Mit der Temperierung wird auch eine minimale Defokussierung der Blutgefäße 19 durch Volumenverkleinerung infolge des sich abkühlenden Eiinhaltes während der Messung der Rückstreustrahlung 26 am geöffneten stumpfen Pol 2 des Eies 1 vermieden. Dazu wird eine Einheit zur Temperierung (nicht eingezeichnet) mit einer temperatureinstellenden Steuereinheit der zu untersuchenden Eier 1 eingesetzt, mit der eine vorgegebene konstante Temperatur zur Vermeidung einer Faltenbildung der inneren Schalenmembran 16 eingestellt wird.

Nach der Geschlechtsbestimmung wird das Loch 4 im stumpfen Pol 2 mittels eines Verschlusses 29 verschlossen und die Bebrütung des als weiblich bestimmten Eies 1 fortgesetzt.

### Ausführungsbeispiel 2

Der im ungeöffneten Ei frei bewegliche Embryo 18 wird durch zweimaliges Kippen eines Vogeleies 1 um einen Winkel von 40° in x- und y- Richtung unter der inneren Schalenmembran 16 zentriert. Eine Öffnung mit einem Durchmesser von 10mm wird am stumpfen Pol 2 am Tag 3,5 der Inkubation eingebracht. Das Loch 4 am stumpfen Pol 2 des Vogeleies 1 wird mittels einer Locherzeugungseinheit (nicht dargestellt) erzeugt, wobei das Loch 4 die Kalkschale 14 und die äußere, eine Luftkammer 17 mit einer inneren Schalenmembran 16 bildenden Schalenmembran 15 betrifft und die innere Schalenmembran 16 unversehrt bleibt.

Im Bereich des Lochs 4 am stumpfen Pol 2 unterhalb der unversehrten inneren Schalenmembran 16 wird dann zumindest ein Blutgefäß 5 registriert und mittels einer einfallenden vorgegebenen Strahlung 30 das Blut darin angeregt, die durch die unversehrte innere Schalenmembran 16 hindurch geführte Rückstreustrahlung 26 gemessen, das Blut zumindest des Blutgefäßes 5 spektroskopisch untersucht und detektiert und für eine Geschlechtsbestimmung ausgewertet, wobei vor und während der Messung eine Temperierung der Eihalterung 6 und der Messumgebung 7 des Eies 1 mit einer zugeordneten Einheit zur Temperierung zur Vermeidung einer anderen und drastischen Abkühlung nach Entnahme aus dem Inkubationsschrank durchgeführt wird. Die Registrierung des Blutgefäßes und Anregung des Blutes wird durch einen Laser mit einer Anregungswellenlänge von 785 nm realisiert. Das Fluoreszenzsignal im Bereich 790-1050 nm wird durch das Hämoglobin des Blutes generiert und ist somit blutspezifisch. Die Positionierung des Laserfokus in das Blutgefäß erfolgt basierend auf der Maximierung der Fluoreszenzintensität, welche mittels des Spektrometers für die Geschlechtsbestimmung gemessen wird.

Nach der Positionierung des Fokus im Blutgefäß erfolgt die Messung der geschlechtsspezifischen Parameter mit einem Spektrometer. Anschließend erfolgt die Datenverarbeitung und Auswertung der membrankorrigierten geschlechtsspezifischen Signale.

Nach der Detektion der gewählten Rückstreustrahlung 26 wird das Loch 4 im Pol 2 mit einem Verschluss 29 verschlossen und die Bebrütung des als weiblich bestimmten Eies 1 wird fortgesetzt, während das als männlich bestimmte Ei aus der Bruthorde 10 aussortiert wird.

### Bezugszeichenliste

- 1: Vogelei
- 2: stumpfer Pol
- 3: spitzer Pol
- 4: Loch im stumpfen Pol
- 5: Blutgefäß
- 6: Eihalterung
- 7: Messumgebung
- 8: Loch am spitzen Pol
- 9: Embryozentriereinheit/zentrale Steuerungseinheit/ Zentriersteuerungseinheit
- 10: Eihorde
- 11: Kippvorrichtung
- 12: vertikale Achse im Ei
- 13: Eischale
- 14: Kalkschale
- 15: äußere Schalenmembran
- 16: innere Schalenmembran
- 17: Luftkammer/Luftblase
- 18: Embryo
- 19: extraembryonale Blutgefäße
- 20: Eiklar
- 21: Dotter
- 22: Dottermembran
- 23: Messstelle/Messfläche
- 24: erste Drehung
- 25: zweite Drehung
- 26: Rückstreustrahlung
- 27: Ramanstrahlung
- 28: Fluoreszenzstrahlung
- 29: Verschluss
- 30: einfallende vorgegebene Strahlung
- 31: Leitung
- 32: Leitung
- 33: erste Dreh-/Kippachse
- 34: Leitung
- 35: Leitung
- 36: zweite Dreh-/Kippachse
- 37: Vorrichtung
- 38: Wölbung

## Patentansprüche

1. Verfahren zur Positionierung einer Messstelle (23) an mindestens einem Blut führenden Gefäß (5, 18, 19) eines geöffneten Vogeleies (1) zur nachfolgenden Geschlechtsbestimmung des Vogeleies (1) mit einer Erzeugung eines Lochs (4) an dem Vogelei (1), das zumindest einen stumpfen Pol (2) und einen spitzen Pol (3) sowie eine Eischale (13) mit einer inneren Schalenmembran (16) und einer äußeren, der Kalkschale (14) des Vogeleies (1) näher liegenden Schalenmembran (15) aufweist, wobei das Vogelei (1) zur Durchführung zumindest einer das Blut betreffenden optischen Messung geöffnet wird,
**dadurch gekennzeichnet,**
**dass** die Erzeugung des Lochs (4) am stumpfen Pol (2) des Vogeleies (1) mittels einer Locherzeugungseinheit durchgeführt wird, wobei das Loch (4) die Kalkschale (14) und die äußere, eine Luftkammer (17) mit einer inneren Schalenmembran (16) bildenden Schalenmembran (15) betrifft und die innere Schalenmembran (16) unversehrt bleibt, wobei im Bereich des Lochs (4) am stumpfen Pol (2) unterhalb der unversehrten inneren Schalenmembran (16) zumindest ein Blutgefäß (5, 19, 18) registriert und mittels einer einfallenden vorgegebenen Strahlung (30) das Blut darin angeregt wird, dessen durch die unversehrte innere Schalenmembran (16) hindurch geführte, das Blut betreffende Rückstreustrahlung (26) gemessen, detektiert und für eine Geschlechtsbestimmung ausgewertet wird,
**dadurch gekennzeichnet,**
**dass** in einer xy-Ebene drehbare/kippbare Achsen (33, 36) mittels einer motorgesteuerten Kippvorrichtung (11), die mit einer zentralen Steuereinheit (9) in Verbindung steht, verdreht werden, sodass bei Abweichung des Embryos (18) aus der zentralen Eiachse (12) der abweichende Embryo (18) in den unmittelbaren Bereich der Eiachse (12) zentriert gebracht wird und ein minimaler Abstand zwischen der Wölbung (38) der inneren Schalenmembran (16) und dem Embryo (18) erreicht wird.

2. Verfahren nach Anspruch 1,
**dadurch gekennzeichnet,**
**dass** bei der Eiöffnung die mit anhaftender äußerer Schalenmembran (15) versehene Kalkschale (14) gelocht wird und das Blut zumindest eines Blutgefäßes (5, 19, 18) spektroskopisch untersucht wird und während der Eiöffnung und der nachfolgenden spektroskopischen optischen Messsignalaufnahme durch mindestens die innere Schalenmembran (16) der Eischale (13) hindurch die innere Schalenmembran (16) unversehrt bleibt.

3. Verfahren nach Anspruch 1 oder 2,
**dadurch gekennzeichnet,**
**dass** vor und während der Messung eine Temperierung der Eihalterung (6) und der Messumgebung (7) des Eies (1) mit einer zugeordneten Einheit zur Temperierung durchgeführt werden.

4. Verfahren nach einem der Ansprüche 1 bis 3,
**dadurch gekennzeichnet,**
**dass** nach der Detektion der gewählten Rückstreustrahlung (26) das Loch (4) im stumpfen Pol (2) mittels eines Verschlusses (29) verschlossen und zumindest die Bebrütung des als weiblich geschlechtsbestimmten Eies (1) fortgesetzt wird.

5. Verfahren nach einem der Ansprüche 1 bis 4, **dadurch gekennzeichnet, dass** zur Registrierung eines Blutgefäßes, optischen Anregungen des Blutes und/oder Messung der das Blut betreffenden Rückstreustrahlung ein oder mehrere Laser eingesetzt werden.

6. Vorrichtung (37) zur Positionierung einer Messstelle (23) an mindestens einem Blut führenden Gefäß (5, 18, 19) eines geöffneten Vogeleies (1) innerhalb einer Eihorde (10) zur nachfolgenden Geschlechtsbestimmung des Vogeleies (1) bei Durchführung des Verfahrens nach einem der Ansprüche 1 bis 5, **dadurch gekennzeichnet,**
**dass** die Vorrichtung (37) zumindest umfasst
- eine Locherzeugungseinheit zur Erzeugung eines Lochs (4) am stumpfen Pol (2) des Vogeleies (1),
- eine optische Messeinrichtung zur Messung der das Blut betreffenden Rückstreustrahlung (26),
- zwei in einer xy-Ebene drehbare/kippbare Achsen (33, 36), die mit der Eihorde (10) in Verbindung stehen, wobei die Achsen (33, 36) von Dreh/Kippsignalen bedient werden,
- eine motorgesteuerte Kippvorrichtung (11), die mit den Achsen (33, 36) in Verbindung steht, und
- eine zentrale Steuerungseinheit (9), die mit der motorgesteuerten Kippvorrichtung (11) in Verbindung steht,
wobei die Achsen (33, 36) derart verdreht werden, dass bei Abweichung des Embryos (18) aus der zentralen Eiachse (12) der abweichende Embryo (18) in den unmittelbaren Bereich der Eiachse (12) zentriert gebracht wird und ein minimaler Abstand zwischen der Wölbung (38) der inneren Schalenmembran (16) und dem Embryo (18) erreicht wird.

7. Vorrichtung nach Anspruch 6,
**dadurch gekennzeichnet,**
**dass** die Verbindungen zwischen der motorgesteuerten Kippvorrichtung (11) und den Achsen (33, 36) zumindest mittels Leitungen (31, 32) und die Verbindungen zwischen der motorgesteuerten Kippvorrichtung (11) und der zentralen Steuerungseinheit (9) zumindest mittels Leitungen (34, 35) ausgebildet sind.

## Claims

1. Method for positioning a measuring point (23) at at least one blood-carrying vessel (5, 18, 19) of an opened bird's egg (1) for subsequent sexing of the bird's egg (1) including a generation of a hole (4) on the bird's egg (1), the latter having at least a blunt pole (2) and a pointed pole (3) and an eggshell (13) with an inner shell membrane (16) and an outer shell membrane (15) located closer to the shell (14) of the bird's egg (1), wherein the bird's egg (1) is opened to carry out at least one optical measurement in relation to the blood, **characterized in that** the hole (4) in the blunt pole (2) of the bird's egg (1) is generated by means of a hole generation unit, wherein the hole (4) relates to the shell (14) and the outer shell membrane (15), which forms an air pocket (17) with an inner shell membrane (16), but the inner shell membrane (16) remains intact, wherein at least one blood vessel (5, 19, 18) is registered below the intact inner shell membrane (16) in the region of the hole (4) in the blunt pole (2) and the blood in said blood vessel is excited by means of incident specified radiation (30), the backscattered radiation (26) thereof relating to the blood and passing through the intact inner shell membrane (16) being measured, detected and evaluated for sexing,
**characterized**
**in that** shafts (33, 36) that are rotatable/tiltable in an xy-plane are twisted by means of a motor-controlled tilt apparatus (11), which is connected to a central control unit (9), so that should the embryo (18) deviate from the central egg axis (12) the deviating embryo (18) is brought into the immediate region of the egg axis (12) in centred fashion and a minimum distance is obtained between the arch (38) of the inner shell membrane (16) and the embryo (18).

2. Method according to Claim 1,
**characterized**
**in that** when the egg is opened the shell (14) provided with the adhering outer shell membrane (15) is perforated and the blood in at least one blood vessel (5, 19, 18) is examined by spectroscopy and the inner shell membrane (16) remains intact while the egg is opened and during the subsequent recording of the spectroscopic optical measurement signal through at least the inner shell membrane (16) of the eggshell (13).

3. Method according to Claim 1 or 2,
**characterized**
**in that** before and during the measurement the egg holder (6) and the measurement surroundings (7) of the egg (1) are temperature-controlled by way of an associated unit for temperature control.

4. Method according to any one of Claims 1 to 3,
**characterized**
**in that** following the detection of the chosen backscattered radiation (26) the hole (4) in the blunt pole (2) is sealed by means of a sealant (29) and at least the incubation of the egg (1) sexed as female is continued.

5. Method according to any one of Claims 1 to 4,
**characterized**
**in that** one or more lasers are used for registering a blood vessel, optically exciting the blood and/or measuring the backscattered radiation relating to the blood.

6. Apparatus (37) for positioning a measurement point (23) at at least one blood-carrying vessel (5, 18, 19) of an opened bird's egg (1) within a horde of eggs (10) for subsequently sexing the bird's egg (1) when carrying out the method according to any one of Claims 1 to 5, **characterized**
**in that** the apparatus (37) at least comprises:
- a hole generation unit for generating a hole (4) in the blunt pole (2) of the bird's egg (1),
- an optical measuring device for measuring the backscattered radiation (26) related to the blood,
- two shafts (33, 36) that are rotatable/tiltable in an xy-plane, which are connected to the horde of eggs (10), wherein the shafts (33, 36) are operated by rotate/tilt signals,
- a motor-controlled tilt apparatus (11), which is connected to the shafts (33, 36), and
- a central control unit (9), which is connected to the motor-controlled tilt apparatus (11),
wherein the shafts (33, 36) are twisted in such a way that should the embryo (18) deviate from the central egg axis (12) the deviating embryo (18) is brought into the immediate region of the egg axis (12) in centred fashion and a minimum distance is obtained between the arch (38) of the inner shell membrane (16) and the embryo (18).

7. Apparatus according to Claim 6,
**characterized**
**in that** the connection between the motor-controlled tilt apparatus (11) and the shafts (33, 36) is formed at least by means of lines (31, 32) and the connections between the motor-controlled tilt apparatus (11) and the central control unit (9) is formed at least by means of lines (34, 35).

## Revendications

1. Procédé pour le positionnement d'un site de mesure (23) au niveau d'au moins un vaisseau (5, 18, 19) guidant le sang d'un œuf de volaille (1) ouvert pour la détermination consécutive du sexe de l'œuf de volaille (1) avec génération d'un trou (4) au niveau de l'œuf de volaille (1), qui présente au moins un gros bout (2) et un bout pointu (3) ainsi qu'une coquille d'œuf (13) pourvue d'une membrane interne (16) de coquille et d'une membrane externe (15) de coquille plus proche de la coquille calcaire (14) de l'œuf de volaille (1), l'œuf de volaille (1) étant ouvert pour réaliser au moins une mesure optique concernant le sang, **caractérisé en ce que** la génération du trou (4) au niveau du gros bout (2) de l'œuf de volaille (1) est réalisée à l'aide d'une unité de génération de trous, le trou (4) concernant la coquille calcaire (14) et la membrane externe (15) de coquille formant une poche d'air (17) avec une membrane interne (16) de coquille et la membrane interne (16) de coquille restant intacte, au moins un vaisseau sanguin (5, 19, 18) étant repéré dans la zone du trou (4) au niveau du gros bout (2) sous la membrane interne (16) de coquille intacte et le sang dans ledit vaisseau étant excité au moyen d'un rayonnement prédéfini incident (30), dont le rayonnement rétrodiffusé (26) guidé à travers la membrane interne (16) de coquille intacte, concernant le sang, étant mesuré, détecté et évalué pour une détermination du sexe, **caractérisé en ce que** des axes (33, 36) pouvant tourner/basculer dans un plan xy sont tournés au moyen d'un dispositif de basculement (11), commandé par un moteur, lequel dispositif est relié à une unité centrale de commande (9), de telle sorte que, lorsque l'embryon (18) s'écarte par rapport à l'axe central de l'œuf (12), l'embryon (18) écarté est amené de manière centrale dans la zone directe de l'axe de l'œuf (12) et qu'une distance minimale entre la courbure (38) de la membrane interne (16) de coquille et l'embryon (18) est atteinte.

2. Procédé selon la revendication 1, **caractérisé en ce que** lors de l'ouverture de l'œuf, la coquille calcaire (14) pourvue de la membrane externe (15) de coquille adhérente est percée et le sang d'au moins un vaisseau sanguin (5, 19, 18) est analysé par spectroscopie et la membrane interne (16) de coquille reste intacte pendant l'ouverture de l'œuf et l'enregistrement consécutif du signal de mesure spectroscopique optique à travers au moins la membrane interne (16) de la coquille d'œuf (13).

3. Procédé selon la revendication 1 ou 2, **caractérisé en ce qu'**avant et pendant la mesure, une thermorégulation du support d'œuf (6) et de l'environnement de mesure (7) de l'œuf (1) est effectuée à l'aide d'une unité associée de thermorégulation.

4. Procédé selon l'une quelconque des revendications 1 à 3, **caractérisé en ce qu'**après la détection du rayonnement rétrodiffusé (26) choisi, le trou (4) dans le gros bout (2) est fermé au moyen d'un bouchon (29) et au moins l'incubation de l'œuf (1) dont le sexe déterminé est féminin est poursuivie.

5. Procédé selon l'une quelconque des revendications 1 à 4, **caractérisé en ce qu'**un ou plusieurs lasers sont utilisés pour le repérage d'un vaisseau sanguin, pour les excitations optiques du sang et/ou pour la mesure du rayonnement rétrodiffusé concernant le sang.

6. Dispositif (37) pour le positionnement d'un site de mesure (23) au niveau d'au moins un vaisseau (5, 18, 19) guidant le sang d'un œuf de volaille (1) ouvert dans un plateau à alvéoles (10) pour la détermination consécutive du sexe de l'œuf de volaille (1) lors de la réalisation du procédé selon l'une quelconque des revendications 1 à 5, **caractérisé en ce que** le dispositif (37) comprend au moins
- une unité de génération de trous pour générer un trou (4) au niveau du gros bout (2) de l'œuf de volaille (1),
- un dispositif de mesure optique pour mesurer le rayonnement rétrodiffusé (26) concernant le sang,
- deux axes (33, 36) pouvant tourner/basculer dans un plan xy, qui sont associés au plateau à alvéoles (10), les axes (33, 36) étant actionnés par des signaux de rotation/basculement,
- un dispositif de basculement (11), commandé par un moteur, lequel dispositif est associé aux axes (33, 36) et
- une unité centrale de commande (9), qui est reliée au dispositif de basculement (11) commandé par un moteur,
les axes (33, 36) étant tournés de telle sorte que, lorsque l'embryon (18) s'écarte par rapport à l'axe central (12) de l'œuf, l'embryon (18) écarté est amené de manière centrale dans la zone directe de l'axe (12) de l'œuf et qu'une distance minimale entre la courbure (38) de la membrane interne (16) de coquille et l'embryon (18) est atteinte.

7. Dispositif selon la revendication 6, **caractérisé en ce que** les connexions entre le dispositif de basculement (11) commandé par un moteur et les axes (33, 36) sont formées au moins par des lignes (31, 32) et les connexions entre le dispositif de basculement (11) commandé par un moteur et l'unité centrale de commande (9) sont formées au moins par des lignes (34, 35).
